# EUROPÄISCHE PATENTSCHRIFT

(11) **EP 2 769 682 B1**
(45) Veröffentlichungstag und Bekanntmachung des Hinweises auf die Patenterteilung: **07.07.2021**
(21) Anmeldenummer: 14155392.5
(22) Anmeldetag: 17.02.2014
(51) Int. Cl.: A61B 17/00, A61B 17/29, A61B 17/3201, A61B 18/12, A61B 90/00, A61B 18/14

(54) **Endoskopisches Instrument sowie Schaft für ein endoskopisches Instrument**
Endoscopic instrument and shaft for an endoscopic instrument
Instrument endoscopique et tige pour un instrument endoscopique

(30) Priorität: 20.02.2013 DE 102013101651
(43) Veröffentlichungstag der Anmeldung: 27.08.2014
(73) Patentinhaber: Karl Storz SE & Co. KG, 78532 Tuttlingen (DE)
(72) Erfinder: Schneider, Sven, 78532 Tuttlingen (DE); Unger, Tobias, 78532 Tuttlingen (DE)

(56) Entgegenhaltungen:
- EP-A1- 1 400 211
- EP-A1- 1 721 577
- EP-A1- 2 653 117
- EP-A2- 2 399 538
- WO-A1-98/01080
- WO-A1-2008/005433
- DE-A1-102006 034 590
- DE-U1- 9 215 053
- US-A1- 2005 131 396
- US-B2- 7 913 891

## Beschreibung

Die vorliegende Erfindung betrifft einen Schaft für ein starres endoskopisches Instrument, der einen lang erstreckten Außenschaft und ein innerhalb des Außenschafts beweglich anordenbares lang erstrecktes Übertragungselement umfasst. Als endoskopisches Instrument werden hierbei Instrumente für mikroinvasive chirurgische Eingriffe bezeichnet, unabhängig davon, ob dieser Eingriff mit oder ohne endoskopische Begleitung erfolgt. Die Erfindung betrifft ferner ein starres endoskopisches Instrument mit einem lang erstreckten Schaft, einem an einem distalen Ende des Schafts angeordneten Werkzeug und einem an einem proximalen Ende des Schafts angeordneten Handgriff, wobei der Schaft einen lang erstreckten Außenschaft und ein innerhalb des Außenschafts beweglich angeordnetes lang erstrecktes Übertragungselement umfasst und wobei das Werkzeug und der Handgriff mit dem Übertragungselement und dem Außenschaft derart verbunden sind, dass das Werkzeug durch den Handgriff mittels einer Bewegung des Übertragungselements relativ zum Außenschaft betätigbar ist.

Endoskopische Operationstechniken haben sich für eine Vielzahl chirurgischer Eingriffe durchgesetzt. Dabei wird durch eine natürliche oder eine mit Hilfe einer Inzision geschaffene künstliche Körperöffnung ein endoskopisches Instrumentarium, das insbesondere ein Endoskop und ein oder mehrere endoskopische Instrumente umfassen kann, zu einem im Körperinneren gelegenen Operationsgebiet geführt. Endoskopische Instrumente weisen hierfür einen lang erstreckten Schaft auf, an dessen distalem, d.h. benutzerfernen Ende ein Werkzeug zur Durchführung chirurgischer Manipulationen angeordnet ist, das von einem am proximalen, d.h. benutzernahen Ende des Schafts angeordneten Handgriff über ein im Schaft angeordnetes langerstrecktes Übertragungselement betätigt werden kann. Der Handgriff verbleibt bei einem endoskopischen Eingriff außerhalb der Körperöffnung, während der Schaft mit dem Werkzeug durch die Körperöffnung eingeführt wird. Die notwendige Nutzlänge des Schafts, um über den durch die natürliche oder künstliche Körperöffnung geschaffenen Zugangsweg das Werkzeug bis zum Operationsgebiet führen zu können, hängt insbesondere von der Art des chirurgischen Eingriffs und von dem genutzten Zugangsweg ab, insbesondere von der Länge des Zugangswegs zwischen der natürlichen oder künstlich geschaffenen Körperöffnung und dem Operationsgebiet. Ferner hängt von der Art des Eingriffs und dem hierfür genutzten Zugangsweg ab, ob ein starres, ein semi-flexibles oder ein flexibles endoskopisches Instrumentarium zum Einsatz kommt.

Bei laparoskopischen Operationen, aber auch bei anderen endoskopischen Eingriffen, werden in erster Linie starre endoskopische Instrumente verwendet. Bei diesen ist der lang erstreckte Schaft im Wesentlichen starr oder nur in geringem Maße flexibel ausgebildet. Während für laparoskopische Eingriffe in der Regel eine Nutzlänge von maximal ca. 500 mm ausreichend ist, kann in einzelnen Fällen, beispielsweise bei adipösen Patienten, eine zum Teil wesentlich größere Nutzlänge erforderlich sein.

Starre endoskopische Instrumente sind häufig für eine mehrfache Verwendung vorgesehen. Nach einem erfolgten Eingriff ist daher vor einer erneuten Verwendung eine Reinigung und Sterilisation des benutzten endoskopischen Instruments erforderlich. Hierfür ist es bekannt, endoskopische Instrumente zerlegbar auszubilden, wobei insbesondere der Handgriff vom Schaft getrennt werden kann; ferner kann ein Schafteinsatz, der das Werkzeug und das Übertragungselement umfasst, aus einem Außenschaft des Schafts entnehmbar ausgebildet sein. Der Handgriff, der Außenschaft und der Einsatz können sodann in an sich bekannter Weise in einer chirurgischen Spülmaschine gereinigt und in einem Autoklav sterilisiert werden. Da der verfügbare Raum in derartigen Reinigungs- und Sterilisationsapparaten begrenzt ist, ist die Reinigung und Sterilisation von Instrumenten mit einer überlangen Nutzlänge, insbesondere mit einer Länge von mehr als 500 mm, auf diese Weise nicht oder nur mit erheblichem Aufwand möglich, beispielsweise kann eine manuelle Reinigung und eine Sterilisation in einem speziell angepassten Autoklav erforderlich sein.

Aus dem Stand der Technik, EP 2 399 538 A2, US 2005/013196 A1, EP 1 400 211 A1 und DE 92 15 053 U1, sind auch Instrumente mit einem starren Schaft und abnehmbaren Endeffektoren bekannt, die Endeffektoren weisen einen Grundkörper auf der mit dem distalen Ende des Schaftes verbunden wird.

Es ist Aufgabe der vorliegenden Erfindung, einen Schaft für ein starres endoskopisches Instrument anzugeben, das zur Verwendung bei einem besonders langen endoskopischen Zugangsweg geeignet ist und bei dem die Reinigung und Sterilisation in einfacherer Weise möglich ist. Ferner ist es Aufgabe der vorliegenden Erfindung, ein derartiges endoskopisches Instrument zu schaffen.

Diese Aufgabe wird durch einen Schaft für ein endoskopisches Instrument gemäß Anspruch 1 sowie durch ein endoskopisches Instrument gemäß Anspruch 12 gelöst. Vorteilhafte Weiterbildungen der Erfindung ergeben sich aus den Unteransprüchen.

Ein erfindungsgemäßer Schaft ist als Schaft für ein starres endoskopisches Instrument insbesondere zur Durchführung endoskopischer chirurgischer Eingriffe ausgebildet. Der erfindungsgemäße Schaft ist zur Einführung in einen körperinneren Hohlraum durch einen endoskopischen Zugangsweg lang erstreckt ausgebildet und umfasst einen lang erstreckten, im Wesentlichen starren Außenschaft und ein ebenfalls lang erstrecktes Übertragungselement, wobei der Außenschaft und das Übertragungselement derart ausgebildet sind, dass das Übertragungselement innerhalb des Außenschafts beweglich angeordnet werden kann. Ferner sind der Außenschaft und das Übertragungselement derart ausgebildet, dass mit einem distalen Endbereich des Schafts ein Werkzeug und mit einem proximalen Endbereich des Schafts ein Handgriff derart verbunden oder verbindbar ist, dass das Werkzeug durch Betätigung des Handgriffs betätigt werden kann. Hierfür sind der Außenschaft und das Übertragungselement an ihren jeweiligen distalen Enden mit dem Werkzeug und an ihren jeweiligen proximalen Enden mit dem Handgriff verbunden oder verbindbar, so dass durch eine Betätigung des Handgriffs das Übertragungselement relativ zum Außenschaft bewegt wird und durch die Bewegung des Übertragungselements das Werkzeug betätigt wird. Vorzugsweise ist der Handgriff mit dem proximalen Ende des Schafts lösbar verbindbar. Hierfür kann ein proximaler Endbereich des Außenschafts und des Übertragungselements ausgebildet sein wie in EP 0 688 187 B1 und DE 197 22 062 A1 offenbart.

Der Außenschaft kann unmittelbar in eine Körperöffnung einführbar sein oder etwa zur Einführung mittels eines Trokars ausgebildet sein.

Das Übertragungselement kann starr oder flexibel ausgebildet sein. Vorzugsweise ist das Übertragungselement als relativ zum Außenschaft in einer Längsrichtung des Schafts verschiebbare Zugstange ausgebildet, die Zugkräfte und zumindest in begrenztem Maße auch Schubkräfte übertragen kann. Das Übertragungselement kann in bestimmten Ausführungen auch Torsionskräfte übertragen. Durch eine Betätigung des Handgriffs kann das Übertragungselement bzw. die Zugstange relativ zum Außenschaft in der Längsrichtung verschoben und dadurch ein bewegliches Teil des Werkzeugs bewegt werden. Erfindungsgemäß umfasst der Außenschaft einen proximalen und einen distalen Abschnitt, die lösbar miteinander verbindbar sind. Der distale Abschnitt ist mit dem Werkzeug und der proximale Abschnitt mit dem Handgriff verbunden oder verbindbar. Der proximale und der distale Abschnitt sind beide jeweils im Wesentlichen starr ausgebildet.

Erfindungsgemäß sind die beiden Abschnitte etwa gleich lang und weisen jeweils etwa die halbe Länge des Außenschafts auf.

Dadurch, dass der Außenschaft in zwei miteinander verbindbare und voneinander trennbare Längsabschnitte aufgeteilt ist, wird eine Zerlegung des Instruments ermöglicht, wodurch die Reinigung bzw. Sterilisation vereinfacht wird. Ein derartiger zerlegbarer Aufbau des Außenschafts ermöglicht eine Reinigung und Sterilisation auch eines endoskopischen Instruments mit überlanger Nutzlänge in Reinigungs- bzw. Sterilisationsapparaten, die für Instrumente einer geringeren Nutzlänge ausgelegt sind. Hierfür ist es vorzugsweise vorgesehen, dass die lösbar miteinander verbindbaren Abschnitte des Außenschafts jeweils eine Länge aufweisen, die maximal der Länge des Schafts von Standardinstrumenten entspricht, insbesondere maximal 500 mm. Ein solcher proximaler und distaler Abschnitt des Außenschafts können daher in für Standardinstrumente ausgelegten Reinigungs- bzw. Sterilisationsapparaten aufbereitet und in für Standardinstrumente dimensionierten Standardsieben aufbewahrt werden.

Vorzugsweise weisen der proximale Abschnitt des Außenschafts an seinem distalen Ende und der distale Abschnitt des Außenschafts an seinem proximalen Ende jeweils ein Verbindungselement zur lösbaren Verbindung miteinander auf, wodurch die miteinander verbundenen Abschnitte des Außenschafts einen starren Außenschaft entsprechend größerer Länge bilden. Die Verbindungselemente können mit dem jeweiligen Abschnitt des Außenschafts fest verbunden oder einstückig mit diesem ausgebildet sein. Die Verbindungselemente können jeweils als axial gerichteter Vorsprung ausgebildet sein oder einen solchen aufweisen, wobei die Vorsprünge des proximalen und des distalen Abschnitts des Außenschafts derart komplementär zueinander ausgebildet sind, dass sie ineinandergreifen können. Insbesondere weisen die Vorsprünge jeweils in axialer Richtung eine Hinterschneidung auf, wobei die Hinterschneidungen der Vorsprünge zum Ineinandergreifen komplementär zueinander ausgebildet sind. Hierdurch kann eine sichere kraft- oder formschlüssige Verbindung erreicht werden.

In besonders vorteilhafter Weise weisen das dem proximalen Abschnitt des Außenschafts und das dem distalen Abschnitt des Außenschafts zugeordnete Verbindungselement jeweils eine komplementär ineinandergreifende Helix auf. Jedes Verbindungselement kann auch eine Mehrzahl von Helices aufweisen, die komplementären Helices des anderen Verbindungselements zusammenwirken. Die Verbindungselemente können jeweils mindestens einen als zueinander komplementäre Helix ausgebildeten Vorsprung aufweisen, oder es kann mindestens bei einem der Verbindungselemente auf der Innen- oder Außenseite eines axial gerichteten Rohrs eine Helix ausgebildet sein, mit der eine komplementäre Helix des anderen Verbindungselements zusammenwirkt. Insbesondere bildet die distale Kante des Vorsprungs des distalen Abschnitts des Außenschafts eine Schraubenlinie, und die proximale Kante des Vorsprungs des proximalen Abschnitts bildet ebenfalls eine Schraubenlinie, wobei beide Schraubenlinien vorzugsweise dieselbe Steigung aufweisen. Auch die der Kante, die die Schraubenlinie bildet, gegenüberliegende Kante des Vorsprungs kann eine Schraubenlinie darstellen. Dadurch, dass die Verbindungselemente des distalen und des proximalen Abschnitts des Außenschafts jeweils eine komplementär ineinandergreifende Helix aufweisen, kann eine einfache und sichere Montage und Zerlegung des Außenschafts ermöglicht werden. Ferner wird hierdurch eine Ausgestaltung der Verbindungselemente ermöglicht, die weder zu einer Erhöhung des Außendurchmessers des Außenschafts noch zu einer wesentlichen Einschränkung des nutzbaren Innenraums innerhalb des Außenschafts führt.

Vorzugsweise ist die Helix des proximalen Abschnitts des Außenschafts im Wesentlichen zwischen den in distaler Richtung verlängerten inneren und äußeren Hüllflächen des proximalen Abschnitts des Außenschafts angeordnet, und die Helix des distalen Abschnitts des Außenschafts ist zwischen den in proximaler Richtung verlängerten inneren und äußeren Hüllflächen des distalen Abschnitts des Außenschafts angeordnet. Dadurch, dass im Bereich der Verbindungselemente weder der Außendurchmesser des Außenschafts vergrößert ist noch der Innendurchmesser wesentlich verringert ist, wird die Einführung des Schafts durch den endoskopischen Zugangsweg erleichtert, und ebenso kann eine Einschränkung der Beweglichkeit des Übertragungselements innerhalb des Außenschafts vermieden werden.

Wenngleich als bevorzugt konstant beschrieben, können die beiden Helices auch nicht konstante, also sich in Axialrichtung verkleinernde wie auch vergrößernde Steigungen aufweisen. Insbesondere sich verkleinernde, das heißt, sich vom zylindrischen Grundschaft in Richtung Helix-Enden hin von eher axial ausgerichtet zu eher tangential ausgerichtet hin verändern. Damit die "geometrisch" gelöst werden kann, das heißt ohne Spalten im zusammengesetzen Schaft, müssen sich hier die Helices in Axialrichtung vom zylindrischen Grundschaft aus verjüngen - und entsprechend die Helixaufnahmen vergrößern. Selbstverständlich können sich die Helices - in Axialrichtung vom zylindrischen Grundschaft aus - verjüngen, auch ohne dass sich ihre Steigung ändert, also auch m it konstanter Steigung.

Gemäß einer bevorzugten Ausführungsform der Erfindung umfasst das Übertragungselement einen proximalen und einen distalen Abschnitt, die lösbar zur Übertragung von in Längsrichtung wirkenden Kräften miteinander verbindbar sind. Der proximale Abschnitt des Übertragungselements ist ferner zur lösbaren Verbindung mit dem Handgriff ausgebildet, und der distale Abschnitt des Übertragungselements ist mit dem Werkzeug verbindbar oder verbunden; der distale Abschnitt des Übertragungselements kann über das Werkzeug mit dem distalen Abschnitt des Außenschafts beweglich, jedoch untrennbar verbunden sein. Der proximale und der distale Abschnitt des Übertragungselements können hinsichtlich ihrer jeweiligen Länge näherungsweise der Länge des proximalen bzw. des distalen Abschnitts des Außenschafts entsprechen. Insbesondere weisen der proximale und der distale Abschnitt jeweils etwa die Hälfte der Länge des Übertragungselements aufweisen. Vorzugsweise weist der distale Abschnitt des Übertragungselements eine derartige Länge auf, dass dieser dann, wenn das Werkzeug mit dem distalen Abschnitt des Übertragungselements und dem distalen Abschnitt des Außenschafts verbunden ist, der distale Abschnitt des Übertragungselements aus dem proximalen Ende des distalen Abschnitts des Außenschafts in proximaler Richtung herausragt. Entsprechend kann der proximale Abschnitt des Übertragungselements eine solche Länge aufweisen, dass das proximale Ende des Übertragungselements aus dem proximalen Ende des Außenschafts in proximaler Richtung herausragt, wenn der proximale und der distale Abschnitt des Außenschafts und des Übertragungselements miteinander verbunden sind. Dadurch, dass das Übertragungselement einen proximalen und einen distalen Abschnitt aufweist, die miteinander verbindbar und voneinander trennbar sind, wird die Reinigung und/oder Sterilisation des Schafts weiter vereinfacht, insbesondere wenn das Übertragungselement starr ausgebildet ist.

Weiterhin ist es bevorzugt, dass der proximale und der distale Abschnitt des Übertragungselements jeweils ein Verbindungselement aufweisen, die eine lösbare, formschlüssige Verbindung des distalen und des proximalen Abschnitts miteinander ermöglichen. Die Verbindungselemente des proximalen und des distalen Abschnitts des Übertragungselements können beispielsweise einstückig mit dem jeweiligen Abschnitt ausgebildet sein oder fest mit diesem verbunden sein. Dadurch, dass eine formschlüssige Verbindung zwischen dem distalen und dem proximalen Abschnitt des Übertragungselements geschaffen werden kann, wird auf einfache Weise eine sichere Verbindung zur Übertragung hoher Kräfte zur Betätigung des Werkzeugs ermöglicht.

Gemäß einer besonders bevorzugten Ausführungsform der Erfindung sind das dem proximalen Abschnitt des Übertragungselements und das dem distalen Abschnitt des Übertragungselements zugeordnete Verbindungselement derart miteinander verbindbar, dass diese dann, wenn die Verbindungselemente innerhalb des Außenschafts bzw. eines Abschnitts des Außenschafts angeordnet sind, unlösbar miteinander verbunden sind und somit auch der proximale und der distale Abschnitt des Übertragungselements unlösbar miteinander verbunden sind. Wenn die Verbindungselemente des Übertragungselements nicht vom Außenschaft umschlossen sind, können die Verbindungselemente ohne Kraftausübung voneinander trennbar sein oder lediglich kraftschlüssig miteinander verbunden sein. Hierzu kann beispielsweise ein Formschlusselement vorgesehen sein, das durch eine Querbewegung eine formschlüssige Verbindung zwischen den Verbindungselementen der beiden Abschnitte des Übertragungselements bewirkt und das durch den Außenschaft am Freigeben der formschlüssigen Verbindung gehindert ist, insbesondere indem eine Querbewegung des Formschlusselements durch den Außenschaft gesperrt ist. Das Formschlusselement kann Teil eines der Verbindungselemente der Abschnitte des Übertragungselements sein oder zusätzlich zu diesen einsetzbar sein. Hierdurch wird eine einfache Montage und Zerlegung des Schafts bei einer besonders sicheren Übertragung der zur Betätigung des Werkzeugs notwendigen Kräfte ermöglicht. Ferner kann hierdurch eine unbeabsichtigte Trennung des proximalen und des distalen Abschnitts des Übertragungselements im zusammengebauten Zustand sicher verhindert werden.

In vorteilhafter Weise sind die Verbindungselemente des proximalen und des distalen Abschnitts des Übertragungselements zur Herstellung einer Schnappverbindung mit mindestens einer bzw. einem, vorzugsweise zwei gelenkig oder elastisch federnd gelagerten Backen bzw. Widerhaken ausgebildet. Die gelenkig oder federnd gelagerten Backen bzw. Widerhaken können dem proximalen oder dem distalen Abschnitt des Übertragungselements zugeordnet sein, wobei der jeweils andere Abschnitt eine mit den Backen bzw. Widerhaken zusammenwirkende Hinterschneidung aufweist. Die Verbindung kann insbesondere formschlüssig ausgebildet sein. Eine Außenkontur der Backen bzw. Widerhaken kann dabei derart ausgebildet sein, dass eine Freigabe der Verbindung durch den aufgesetzten Außenschaft sicher verhindert wird, indem ein zum Öffnen der Verbindung notwendiges Ausweichen der Backen bzw. Widerhaken durch den Außenschaft gesperrt wird. Eine derartige Schnappverbindung ist besonders sicher und besonders einfach verbindbar.

Gemäß einer weiteren bevorzugten Ausführungsform der Erfindung sind die Verbindungselemente in einer Richtung quer zur Längsachse des Schafts ineinander einschiebbar ausgebildet, wobei zusätzlich eine Sicherung durch einen quer zur Längsrichtung des Übertragungselements in dieses einsetzbaren Sicherungsstift erfolgen kann. Insbesondere kann die Verbindung formschlüssig sein. Vorzugsweise sind die Verbindungselemente dann, wenn das Übertragungselement im Außenschaft angeordnet ist, unlösbar miteinander verbunden, wobei ein seitliches Auseinanderschieben bzw. ein Entnehmen des Sicherungsstifts zum Lösen der Verbindung formschlüssig durch den Außenschaft gesperrt wird. Auch hierdurch wird eine besonders sichere und einfach herzustellende und lösbare Verbindung geschaffen.

Gemäß einer weiteren bevorzugten Ausführungsform der Erfindung ist eine durch Relativdrehung des distalen Abschnitts zum proximalen Abschnitt des Übertragungselements miteinander verbindbare und voneinander trennbare Verbindung vorgesehen, die beispielsweise als Bajonett- oder Gewindeverbindung ausgebildet ist. Vorzugsweise ist die Gewinderichtung entgegen der Richtung einer Helix, die von miteinander zusammenwirkenden Verbindungselementen des proximalen und des distalen Abschnitts des Außenschafts gebildet wird, gerichtet. Hierdurch wird ebenfalls eine sichere und auf einfache Weise verbindbare und wieder lösbare Verbindung der Abschnitte des Übertragungselements geschaffen.

Gemäß einer besonders bevorzugten Ausführungsform der Erfindung sind der distale und der proximale Abschnitt des Übertragungselements verdrehgesichert miteinander verbindbar, der distale Abschnitt des Übertragungselements ist verdrehgesichert mit dem distalen Abschnitt des Außenschafts verbunden oder verbindbar, und der proximale Abschnitt des Übertragungselements ist verdrehgesichert mit dem proximalen Abschnitt des Außenschafts verbindbar. Eine verdrehgesicherte Verbindung zwischen dem proximalen und dem distalen Abschnitt des Übertragungselements kann insbesondere durch eine entsprechende Ausbildung der dem proximalen und dem distalen Abschnitt zugeordneten Verbindungselemente erreicht werden, beispielsweise durch eine nicht-axialsymmetrische Ausbildung der Verbindungselemente und/oder durch einen in Querrichtung eingesetzten Stift. Hierdurch kann eine axiale Verdrehung der beiden Abschnitte zueinander verhindert werden. Weiter ist der distale Abschnitt des Übertragungselements verdrehgesichert mit dem distalen Abschnitt des Außenschafts verbunden oder verbindbar, beispielsweise durch einen in einer Nut oder in einem Schlitz geführten, quer zur Längsachse des Schafts gerichteten Stift. Ferner ist der proximale Abschnitt des Außenschafts verdrehgesichert mit dem proximalen Abschnitt des Übertragungselements verbunden. Dies kann beispielsweise durch eine nicht-axialsymmetrische Ausbildung des proximalen Abschnitts des Übertragungselements und des proximalen Abschnitts des Außenschafts in ihrem jeweiligen proximalen Endbereich erreicht werden. In besonders vorteilhafter Weise sind der proximale und der distale Abschnitt des Übertragungselements, ebenso wie der proximale und der distale Abschnitt des Außenschafts, jeweils selbst möglichst rotationssteif ausgebildet. Hierdurch wird eine Verdrehsicherung des proximalen relativ zum distalen Abschnitt des Außenschafts erreicht, wodurch ein unbeabsichtigtes Lösen einer durch Relativdrehung lösbaren Verbindung zwischen dem proximalen und dem distalen Abschnitt des Außenschafts sicher verhindert werden kann.

In besonders bevorzugter Weise ist der proximale Abschnitt des Außenschafts bei mit dem Schaft verbundenem Handgriff mit dem proximalen Abschnitt des Übertragungselements verdrehgesichert verbunden. Eine derartige Verbindung ist vorzugsweise wie in EP 0 688 187 B1 beschrieben ausgebildet. Hierdurch ist der weitere besondere Vorteil erzielbar, dass eine Zerlegung eines Schafts, bei dem der Außenschaft durch eine durch Relativdrehung lösbare Verbindung des proximalen mit dem distalen Abschnitt des Außenschafts geschaffen wird, nur nach Trennung des Handgriffs vom Schaft möglich ist und somit eine unbeabsichtigte Zerlegung während einer Operation sicher vermieden werden kann; andererseits ist nach der Operation eine Zerlegung des Instruments auf einfache Weise möglich.

Gemäß einer bevorzugten Ausführungsform der Erfindung ist der Schaft als Schaft für ein elektrochirurgisches endoskopisches Instrument ausgebildet, wobei eine Stromübertragung zum Werkzeug über das Übertragungselement erfolgt, das hierfür metallisch ausgebildet ist und in elektrisch leitender Verbindung mit dem Werkzeug und einem HF-Anschluss steht bzw. in eine solche gebracht werden kann. Insbesondere ist das Werkzeug elektrisch leitend mit dem distalen Abschnitt Übertragungselements verbunden, und der proximale Abschnitt des Übertragungselements ist mit einem an einem Handgriff angeordneten HF-Anschluss elektrisch leitend verbindbar. Auch das Werkzeug besteht vorzugsweise aus einem metallischen Werkstoff. Vorzugsweise ist der Außenschaft von einer Isolierung umgeben, die beispielsweise als Schrumpfschlauch ausgebildet sein kann. Am distalen Ende des proximalen Abschnitts des Außenschafts und/oder am proximalen Ende des distalen Abschnitts des Außenschafts kann ein rohrförmiges isolierendes Zwischenstück angeordnet sein, wodurch die Entstehung einer Kriechstrecke im zusammengebauten Zustand vermieden wird.

Ein erfindungsgemäßes starres endoskopisches Instrument umfasst einen im Wesentlichen starren, lang erstreckten Schaft, ein an einem distalen Ende des Schafts angeordnetes Werkzeug und einen an einem proximalen Ende des Schafts angeordneten Handgriff. Der Schaft umfasst einen lang erstreckten Außenschaft und ein innerhalb des Außenschafts beweglich angeordnetes, lang erstrecktes Übertragungselement. Das Werkzeug und der Handgriff sind mit dem Übertragungselement und dem Außenschaft derart verbunden, dass das Werkzeug durch den Handgriff mittels einer Bewegung des Übertragungselements relativ zum Außenschaft betätigbar ist. Erfindungsgemäß ist der Schaft wie oben beschrieben ausgebildet. Das endoskopische Instrument weist insbesondere eine Überlänge auf und ist zur Verwendung bei besonders langen endoskopischen Zugangswegen geeignet. Insbesondere dadurch, dass der Außenschaft in einen proximalen und einen distalen Abschnitt zerlegbar ist, wird eine Reinigung bzw. Sterilisation in Geräten mit Standardabmessungen ermöglicht.

Das Werkzeug ist insbesondere zur Ausführung chirurgischer Manipulationen in einem durch den Zugangsweg erreichbaren körperinneren Hohlraum ausgebildet und kann hierfür zwei miteinander zusammenwirkende Werkzeugelemente aufweisen. Das Werkzeug kann beispielsweise eine chirurgische Schere oder Greifzange sein, die ein oder zwei bewegliche, relativ zueinander schwenkbare Scheren- bzw. Maulteile aufweist, wobei ein bewegliches Werkzeugelement gelenkig mit einem feststehenden Werkzeugelement verbunden ist, oder es können zwei bewegliche Werkzeugelemente miteinander über ein Gelenk verbunden sein. Das feststehende Werkzeugelement bzw. das Gelenk ist mit einen distalen Endbereich des Außenschafts verbunden und das bzw. die beweglichen Werkzeugelemente etwa über eine Hebelanordnung mit dem Übertragungselement oder umgekehrt. Hierdurch kann in an sich bekannter Weise durch eine Längsverschiebung des Übertragungselements eine Schwenkbewegung der Scheren bzw. Maulteile zueinander bewirkt werden, wie beispielsweise in der Patentschrift DE 10 2006 028 001 B4 offenbart ist.

Das Werkzeug kann aber auch beispielsweise ein axial verschiebbares Werkzeugelement umfassen, das direkt mit dem Übertragungselement verbunden ist. Das Werkzeug kann vom Schaft lösbar sein, oder es kann mit dem distalen Abschnitt des Außenschafts und/oder dem Übertragungselement bzw. dem distalen Abschnitt des Übertragungselements untrennbar, jedoch ggf. gelenkig, verbunden sein.

Vorzugsweise ist der Handgriff vom proximalen Ende des Schafts trennbar und mit diesem wieder verbindbar. Hierfür kann ein proximaler Endbereich des Außenschafts und des Übertragungselements ausgebildet sein wie in EP 0 925 028 B1 bzw. DE 197 22 062 A1 beschrieben. Der Handgriff kann ausgebildet sein, um in der daraus bekannten Weise auf das proximale Ende des proximalen Abschnitts des Außenschafts aufgesetzt zu werden.

Zum Montieren eines endoskopischen Instruments mit einem wie oben beschrieben ausgebildeten Schaft wird insbesondere folgendermaßen vorgegangen:
In einem ersten Schritt wird das Verbindungselement des distalen Abschnitts des Übertragungselements mit dem Verbindungselement des proximalen Abschnitts des Übertragungselements verbunden. Hierdurch entsteht das für die Übertragung von Kräften in Längsrichtung zur Betätigung des Werkzeugs dienende Übertragungselement des endoskopischen Instruments. Wenn der distale Abschnitt des Außenschafts über das Werkzeug mit dem distalen Abschnitt der Zugstange beweglich, jedoch untrennbar verbunden ist, ist somit das Übertragungselement mit dem Werkzeug und dem distalen Abschnitts des Außenschafts verbunden. Im zweiten Schritt wird der proximale Abschnitt des Außenschafts aus proximaler Richtung über den aus dem distalen Abschnitt des Außenschafts nach proximal heraus ragenden Teil des Übertragungselements geschoben. Mit Hilfe der Verbindungselemente des proximalen und des distalen Abschnitts des Außenschafts werden die beiden Abschnitte des Außenschafts miteinander verbunden und bilden nun den durchgehenden, starren Außenschaft des endoskopischen Instruments, in dem das Übertragungselement angeordnet ist. Der Zusammenbau des Schafts ist damit abgeschlossen.

In einem weiteren Schritt wird der Handgriff mit dem Schaft verbunden, indem beispielsweise ein proximaler Endbereich des Außenschafts mit einem daran angeordneten Verbindungsmechanismus in eine rohrförmige Kupplung des Handgriffs eingeschoben wird. Der Handgriff umfasst einen feststehenden und einen beweglichen Teil, wobei durch Bewegen des beweglichen Teils relativ zum feststehenden Teil das Werkzeug betätigt werden kann. Der Handgriff kann weiterhin ein Drehrad zur Drehung des Außenschafts und einer entsprechenden Änderung der Orientierung des Werkzeugs umfassen sowie einen Riegelmechanismus zur zusätzlichen Verriegelung des Außenschafts im Handgriff und einen elektrischen Anschluss für ein HF-Kabel.

Zum Zerlegen des endoskopischen Instruments wird der Außenschaft aus der Kupplung des Handgriffs entnommen, wofür beispielsweise der bewegliche Teil des Handgriffs in eine Demontageposition gebracht und ein Druckknopf des Riegelmechanismus gedrückt wird. Der proximale Abschnitt des Außenschafts wird durch Trennen der Verbindungselemente des Außenschafts von dem distalen Abschnitt getrennt und über das Übertragungselement in proximaler Richtung abgezogen. Durch Trennen der Verbindungselemente des Übertragungselements werden der distale und der proximale Abschnitt des Übertragungselements voneinander gelöst. Die einzelnen Elemente des endoskopischen Instruments haben jeweils eine geringere Länge als die Gesamtlänge des zusammengebauten Instruments, erfindungsgemäß nur die halbe Länge, und können in einem Standard-Sieb aufbewahrt und in Standard-Reinigungs- bzw. Sterilisationsapparaten gereinigt bzw. sterilisiert werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen, der Rahmen der Erfindung wird durch die Ansprüche definiert.

Weitere Aspekte der Erfindung ergeben sich aus der nachfolgenden Beschreibung eines bevorzugten Ausführungsbeispiels und der beigefügten Zeichnung. Es zeigen:
Fig. 1 einen erfindungsgemäßen Schaft mit einem an dessen distalem Ende angeordneten Werkzeug in Seitenansicht;
Fig. 2a und 2b den proximalen Abschnitt des Außenschafts gemäß Fig. 1 in einem Längsschnitt bzw. in einer perspektivischen Ansicht;
Fig. 3a und 3b den distalen Abschnitt des Außenschafts mit dem Werkzeug und dem distalen Abschnitt des Übertragungselements in einem Längsschnitt bzw. einer Seitenansicht;
Fig. 4a bis 4c den proximalen Abschnitt des Übertragungselements in zwei Seitenansichten und einer perspektivischen Ansicht;
Fig. 5 den distalen Abschnitt des Außenschafts mit dem Werkzeug und dem aus seinem distalen und proximalen Abschnitt zusammengesetzten Übertragungselement in einer Seitenansicht;
Fig. 6 den proximalen Endbereich des Schafts mit einem mit diesem verbundenen Handgriff in einer perspektivischen Ansicht;
Fig. 7 bis 9 jeweils im schematischen Längsschnitt drei Ausführungsbeispiele der Verbindungselemente des proximalen und des distalen Abschnitts des Übertragungselements in miteinander verbundenem Zustand;
Fig. 10a und 10b im schematischen Längsschnitt ein weiteres Ausführungsbeispiel der Verbindungselemente des proximalen und des distalen Abschnitts des Übertragungselements in miteinander verbundenem Zustand bzw. eines der Verbindungselemente gemäß Fig. 10a in perspektivischer Ansicht;
Fig. 11 bis 13 jeweils im schematischen Längsschnitt drei weitere Ausführungsbeispiele der Verbindungselemente des proximalen und des distalen Abschnitts des Übertragungselements in miteinander verbundenem Zustand.

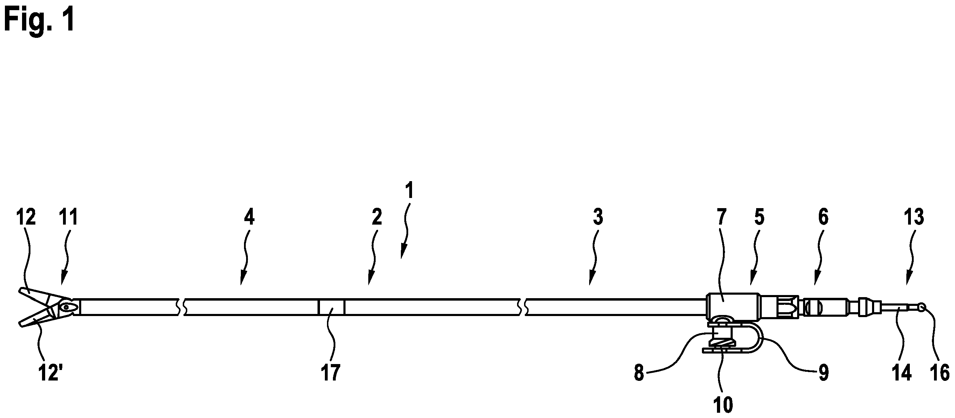

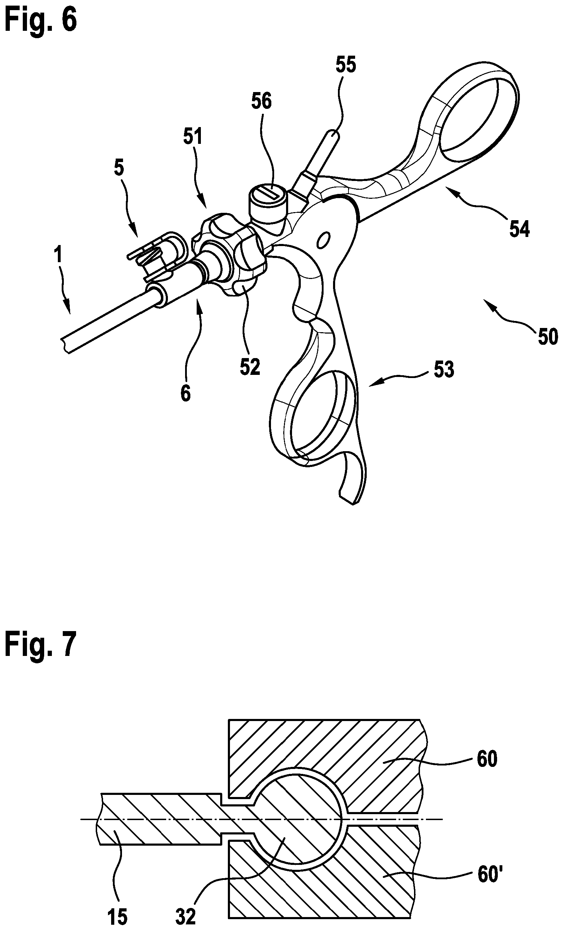

Wie in Fig. 1 beispielhaft dargestellt ist, umfasst ein Schaft 1 für ein endoskopisches Instrument einen langerstreckten Außenschaft 2, der in einen proximalen Abschnitt 3 und einen distalen Abschnitt 4 geteilt ist. Der proximale Abschnitt 3 des Außenschafts 2 weist einen Spülanschluss 5 sowie einen Verbindungsmechanismus 6 zur Verbindung mit einem Handgriff auf. Der Spülanschluss 5 umfasst eine Hülse 7, an der ein Anschlussstutzen 8 ansetzt, der zum Anschluss eines Spül- bzw. Saugschlauchs mit Hilfe eines Luer-Lock ausgebildet ist und durch einen über eine Lasche 9 unverlierbar befestigten Stopfen 10 verschlossen werden kann. Der Verbindungsmechanismus ist derart ausgebildet, dass ein Handgriff 50 drehbar angesetzt werden kann und über ein mit dem proximalen Endbereich des proximalen Abschnitts 3 des Außenschafts 2 drehfest verbindbares Drehrad 52 der Außenschaft 2 relativ zum Handgriff um die Längsachse des Außenschafts 2 gedreht werden kann (s. Fig. 6). Mit dem distalen Abschnitt 4 des Außenschafts 2 ist ein Werkzeug 11 verbunden, das im dargestellten Ausführungsbeispiel als Schere mit zwei relativ zum distalen Abschnitt 4 des Außenschafts 2 schwenkbaren Scherenblättern 12, 12' ausgebildet ist.

Innerhalb des Außenschafts 2 ist eine Zugstange 13 in Längsrichtung des Außenschafts 2 verschiebbar angeordnet. Wie unten näher beschrieben wird, umfasst die Zugstange 13 einen proximalen Abschnitt 14 und einen distalen Abschnitt 15 (in Fig. 1 nicht erkennbar), wobei der proximale Abschnitt 14 der Zugstange 13 im Wesentlichen innerhalb des proximalen Abschnitts 3 des Außenschafts 2 und der distale Abschnitt 15 der Zugstange 13 im Wesentlichen innerhalb des distalen Abschnitts 4 des Außenschafts 2 angeordnet ist. Das proximale Ende des proximalen Abschnitts 14 der Zugstange 13 wird durch ein Verbindungselement, beispielsweise eine Kugel 16 gebildet, die mit einem beweglichen Teil des Handgriffs verbunden werden kann, um durch Betätigen des beweglichen Teils die Zugstange 13 in Längsrichtung zu verschieben. Die Zugstange 13 ist dabei derart ausgebildet, dass sie sowohl Zug- als auch Schubkräfte in Längsrichtung des Schafts 1 übertragen kann. Der distale Endbereich des in Fig. 1 nicht erkennbaren distalen Abschnitts 15 der Zugstange 13 ist über einen Hebelmechanismus mit den beweglichen Scherenblättern 12, 12' derart verbunden, dass durch Verschieben der Zugstange 13 in distale Richtung die Scherenblätter 12, 12' geöffnet und durch Verschieben in proximale Richtung geschlossen werden können. Der distale Endbereich des proximalen Abschnitts 3 des Außenschafts 2 wird durch eine Isolierhülse 17 gebildet. Der Schaft 1 weist eine Überlänge auf und ermöglicht eine Nutzlänge von beispielsweise mehr als 500 mm.

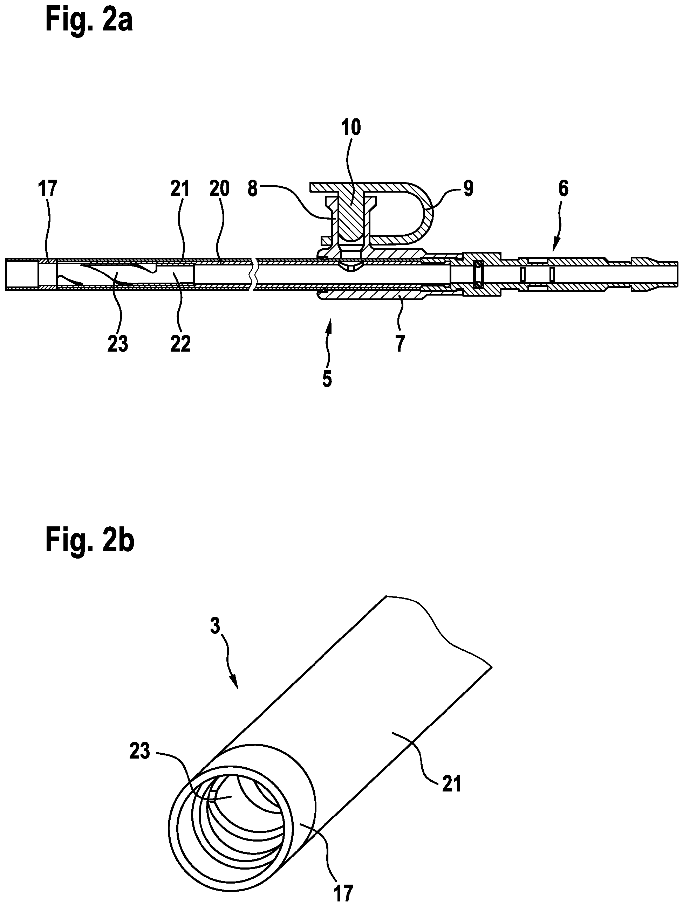

Wie in Fig. 2a in einem Längsschnitt gezeigt, umfasst der Außenschaft ein metallisches Rohr 20, das von einer Isolierung, die etwa durch einen auf das Rohr 20 aufgeschrumpften Schrumpfschlauch 21 gebildet wird, umhüllt ist. Im distalen Endbereich des Rohrs 20 ist in dieses ein rohrförmiger Einsatz 22 eingesetzt und fest mit dem Rohr 20 verbunden, der im distalen Bereich in Form einer ersten Helix 23 ausgebildet ist. Im Bereich des Einsatzes 22 ist der durchgehende Hohlraum des proximalen Abschnitts 3 des Außenschafts 2 höchstens geringfügig eingeengt. Das distale Ende des proximalen Abschnitts 3 des Außenschafts 2 wird durch die Isolierhülse 17 gebildet, die mit dem Einsatz 22 und dem Rohr 20 fest verbunden ist und aus einem Kunststoffmaterial besteht. Im proximalen Endbereich weist der proximale Abschnitt 3 des Außenschafts 2 einen Spülanschluss 5 zum Anschließen einer Spül- bzw. Saugeinrichtung sowie einen Verbindungsmechanismus 6 zum Verbinden mit einem Handgriff auf.

In Fig. 2b ist der distale Endbereich des proximalen Abschnitts des Außenschafts 2 in einer schräg aus distaler Richtung gesehenen perspektivischen Ansicht dargestellt. Hierbei ist die distalseitig an den Schrumpfschlauch 21 anschließende Isolierhülse 17 zu erkennen, innerhalb derer ein Teil der ersten Helix 23 sichtbar ist. Dabei sind runde Konturen aus zeichnungstechnischen Gründen als Polygonzüge dargestellt.

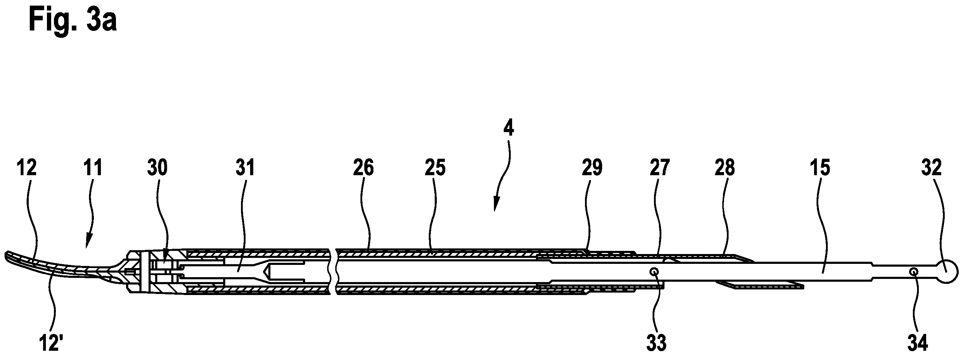

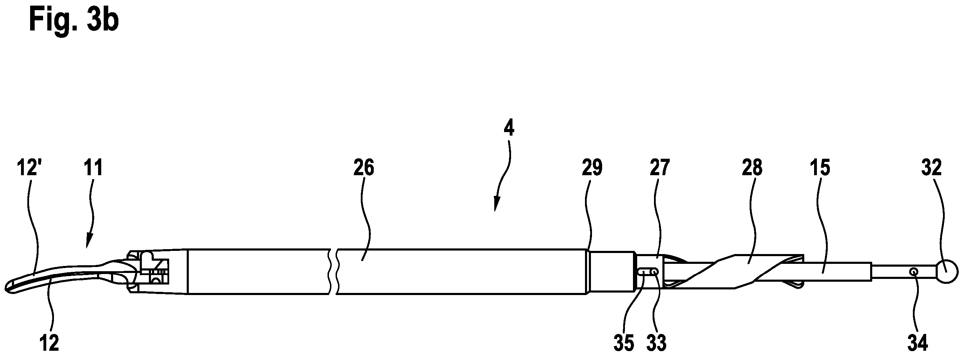

In den Figuren 3a und 3b ist der distale Abschnitt 4 des Außenschafts 2 mit dem Werkzeug 11 und dem im distalen Abschnitt 4 des Außenschafts 2 längsverschiebbar angeordneten distalen Abschnitt 15 der Zugstange 13 gezeigt. Der distale Abschnitt 4 des Außenschafts 2 umfasst ein insbesondere metallisches Rohr 25, das von einer Isolierung, etwa einem Schrumpfschlauch 26, umschlossen wird. Der Schrumpfschlauch 26 bildet über der proximalen Kante des Rohrs 25 einen Absatz 29. Im proximalen Endbereich des Rohrs 25 ist in dieses ein rohrförmiger Einsatz 27 eingesetzt und fest mit dem Rohr 25 verbunden, der proximalseitig in eine zweite Helix 28 ausläuft. Der Innenraum des distalen Abschnitts 4 des Außenschafts ist im Bereich des Einsatzes 27 nur geringfügig eingeschränkt. Der Einsatz 27 mit der zweiten Helix 28 weist einen Außendurchmesser auf, der näherungsweise dem Innendurchmesser des Rohrs 20 des proximalen Abschnitts des Außenschafts 3 in dessen distalem Endbereich sowie dem Innendurchmesser in einem Abschnitt der Isolierhülse 17 entspricht. Die zweite Helix 28 ist derart komplementär zu der ersten Helix 23 ausgebildet, dass diese durch eine schraubenförmige Bewegung des distalen Abschnitts 4 relativ zum proximalen Abschnitt 3 des Außenschafts 2 mit einer Rotation um ca. 360° miteinander in Eingriff gebracht werden können.

Wenngleich in dem obigen Ausführungsbeispiel ca. 360° betragend, kann die Verbindung der beiden Helices auch um andere, geringere wie auch größere Winkel erfolgen. Zudem muss die Steigung der Helices in Axialrichtung gesehen nicht konstant sein, sondern könnte sich verändern, insbesondere stetig verändern, insbesondere sich verkleinern, das heißt vom zylindrischen Grundschaft in Richtung Helix-Enden hin sich von eher axial ausgerichtet zu eher tangential ausgerichtet hin verändern. Damit die "geometrisch" gelöst werden kann, das heißt ohne Spalten im zusammengesetzen Schaft, müssen sich hier die Helices in Axialrichtung vom zylindrischen Grundschaft aus verjüngen - und entsprechend die Helixaufnahmen vergrößern. Selbstverständlich können sich die Helices - in Axialrichtung vom zylindrischen Grundschaft aus - verjüngen, auch ohne dass sich ihre Steigung ändert, also auch mit konstanter Steigung.

Der Innen- und der Außendurchmesser des Einsatzes 27 des distalen Abschnitts 4 des Außenschafts 2 mit der zweiten Helix 28 entspricht dabei im Wesentlichen dem Innen- bzw. Außendurchmesser des Einsatzes 22 des proximalen Abschnitts 3 des Außenschafts 2 mit der ersten Helix 23.

Wie in Fig. 3a und 3b zu erkennen ist, sind die Scherenblätter 12, 12' des Werkzeugs 11 über einen Hebelmechanismus 30 mit dem distalen Endbereich 31 des distalen Abschnitts 15 der Zugstange 13 verbunden. Der distale Abschnitt 15 der Zugstange 13 trägt an seinem proximalen Ende ein Verbindungselement zum Verbinden mit dem proximalen Abschnitt 14 der Zugstange 13, wobei das Verbindungselement als Kugel 32 ausgestaltet ist. Weiterhin weist der distale Abschnitt 15 der Zugstange 13 in seinem proximalen Endbereich einen ersten Stift 33 und einen zweiten Stift 34 auf, die jeweils quer zur Längsachse der Zugstange 13 gerichtet sind und wobei der erste Stift 33 in den in axialer Richtung verlaufenden Schlitz 35 des Einsatzes 27 hineinragt und in diesem in Längsrichtung geführt ist. Die Länge des Schlitzes 35 erlaubt eine zum Betätigen des Werkzeugs 11 ausreichende Längsverschiebung der Zugstange 13.

Wie in Fig. 3a angedeutet ist, ist der vom distalen Abschnitt 4 des Außenschafts 2 gebildete, bis in die Nähe des distalen Endes des Außenschafts 2 axial durchgehende Hohlraum derart ausgebildet, dass der distale Abschnitt 15 des Übertragungselements 13 darin mit Spiel geführt ist, wodurch ein Zwischenraum zwischen der Außenseite des Übertragungselements 13 und der Innenseite des Außenschafts 2 verbleibt. Dieser Zwischenraum kann zur Durchleitung einer Spülflüssigkeit bzw. einer Reinigungsflüssigkeit genutzt werden. Aus im proximalen Abschnitt 3 des Außenschafts 2 ist ein solcher Zwischenraum vorhanden, der mit dem Zwischenraum des distalen Abschnitts 4 und mit dem Anschlussstutzen 8 verbunden ist.

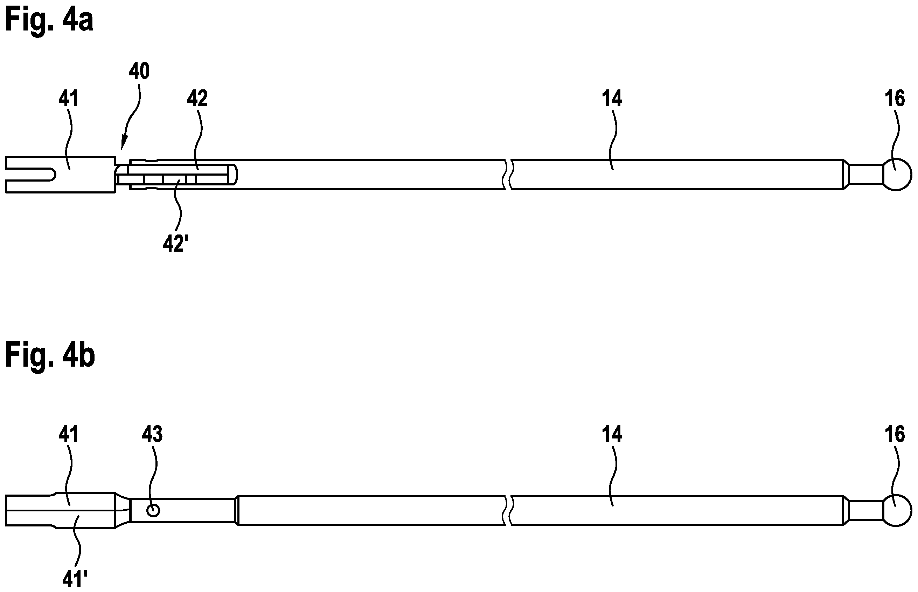

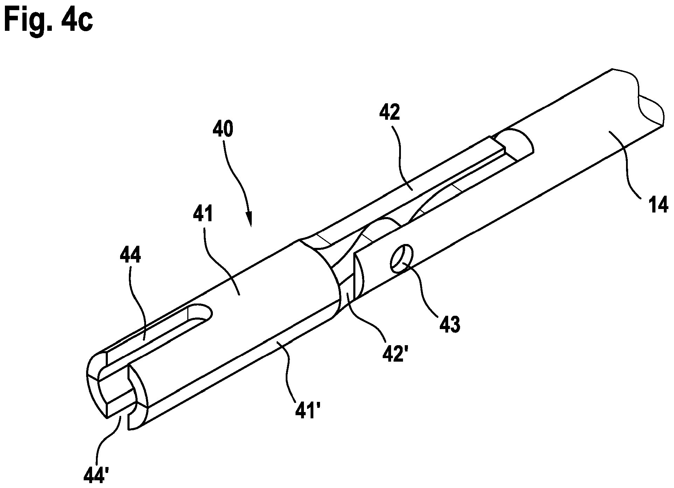

In den Figuren 4a bis 4c ist der proximale Abschnitt 14 der Zugstange 13 in zwei um 90° gegeneinander gedrehten Seitenansichten (Fig. 4a, 4b) sowie in einer schräg aus distaler Richtung gesehenen perspektivischen Ansicht (Fig. 4c) gezeigt. An seinen proximalen Ende weist der proximale Abschnitt 14 der Zugstange 13 ein als Kugel 16 ausgebildetes Verbindungselement zum Verbinden mit einem beweglichen Teil des Handgriffs auf. An seinem distalen Ende weist der proximale Abschnitt 14 der Zugstange 13 ein Verbindungselement zur Verbindung mit dem Verbindungselement des distalen Abschnitts 15 der Zugstange 13 auf, der, wie in den Fig. 4a bis 4c gezeigt, als Schnappmechanismus 40 zur Aufnahme der Kugel 32 des distalen Abschnitts 15 der Zugstange 13 ausgebildet ist. Der Schnappmechanismus 40 umfasst zwei miteinander zusammenwirkende Backen 41, 41', die bei geschlossener Stellung einen im wesentlichen zylindrischen Innenraum in Form eines axial gerichteten Sacklochs umschließen, dessen proximaler Endbereich eine Erweiterung zur Aufnahme der Kugel 32 aufweist (in den Fig. 4a bis 4c nicht erkennbar). Die Backen 41, 41' sind jeweils mit einer Lasche 42, 42' an einem durch die Bohrung 43 geführten, in Fig. 4a bis 4c nicht gezeigten, Stift schwenkbar gelagert. Zumindest die Lasche 42 ist dabei elastisch biegsam ausgebildet und gegen die andere Lasche 42' oder gegen den proximalen Abschnitt 14 der Zugstange elastisch federnd abgestützt, so dass die beiden Backen 41, 41' gegen eine elastische Rückstellkraft geöffnet werden können und von dieser wieder geschlossen werden. Die Backen 41, 41' weisen an ihrem distalen Ende jeweils einen in axialer Richtung verlaufenden, in distaler Richtung offenen Schlitz 44, 44' auf.

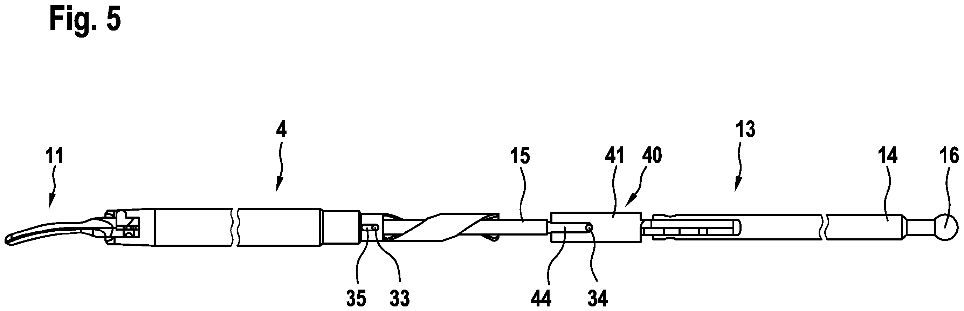

Fig. 5 zeigt den distalen Abschnitt 4 des Außenschafts 2 mit dem Werkzeug 11 und der Zugstange 13, die durch den proximalen Abschnitt 14 und den distalen Abschnitt 15 der Zugstange gebildet wird, die über den Schnappmechanismus 40 miteinander verbunden sind. Dabei ist die Kugel 32, die das am proximalen Ende des distalen Abschnitts 15 der Zugstange 13 angeordnete Verbindungselement bildet, in die Backen 41, 41' des Schnappmechanismus 40 eingeführt und wird von diesen gehalten. Wie in Fig. 5 zu erkennen ist, ragt der zweite Stift 34 des distalen Abschnitts 15 in den Schlitz 44 der Backe 41 hinein und wird in diesem in axialer Richtung verschiebbar geführt. In Fig. 5 ist ebenfalls zu erkennen, dass der erste Stift 33 in dem Längsschlitz 35 des Einsatzes 27 des distalen Abschnitts 4 des Außenschafts 2 in ähnlicher Weise geführt ist.

Der Zusammenbau eines endoskopischen Instruments, das den zuvor beschriebenen Schaft aufweist, erfolgt auf folgende Weise:
Der distale Abschnitt 4 des Außenschafts 2 ist über das Werkzeug 11 und den Hebelmechanismus 30 mit dem distalen Abschnitt 15 der Zugstange verbunden (s. Fig. 3a, 3b). In einem ersten Schritt des Zusammenbaus wird das Verbindungselement des distalen Abschnitts 15 der Zugstange 13, nämlich die Kugel 32 mit dem proximalen Abschnitt 14 der Zugstange 13 verbunden, indem die Kugel 32 zwischen die Backen 41, 41' des Schnappmechanismus 40 eingeführt wird. Dabei wird auch der zweite Stift 34 in die Schlitze 44, 44' eingeführt. Der distale Abschnitt 15 ist nun kraftschlüssig und verdrehgesichert mit dem proximalen Abschnitt 14 der Zugstange verbunden (s. Fig. 5). Im zweiten Schritt wird der proximale Abschnitt 3 des Außenschafts 2 aus proximaler Richtung über den proximalen Abschnitt 14 der Zugstange 13 mit dem Schnappmechanismus 40 sowie über den proximalen Endbereich des distalen Abschnitts 15 der Zugstange 13 geschoben und durch eine schraubenförmige Bewegung die erste Helix 23 des proximalen Abschnitts 3 mit der zweiten Helix 28 des distalen Abschnitts 4 des Außenschafts 2 in Eingriff gebracht. Dadurch greift die Isolierhülse 17 über einen proximalen Endbereich der Schrumpfschlauchs 26 des distalen Abschnitts 4 und liegt näherungsweise an dem Absatz 29. Der Zusammenbau des Schafts 1 gemäß Fig. 1 ist nun abgeschlossen.

In einem weiteren Schritt wird der Handgriff 50 mit dem Schaft 1 verbunden, indem dieser, wie in Fig. 6 durch den Pfeil angedeutet, mit dem Verbindungsmechanismus 6 in eine Kupplung 51 des Handgriffs 50 eingeschoben wird. Hierdurch wird der Schaft 1 derart am Handgriff gehalten, dass der Schaft 1 über ein Drehrad 52 relativ zum Handgriff 50 um seine Längsachse gedreht werden kann und die Kugel 16, die das proximalseitige Verbindungselement der Zugstange 13 bildet, mit dem schwenkbaren Teil 53 des Handgriffs 50 in Eingriff steht. Durch Betätigen des schwenkbaren Teils 53 wird die Zugstange 13 in axialer Richtung verschoben. Der Verbindungsmechanismus 6 und die Kupplung 51 können ferner wie beispielsweise in EP 0 688 187 B1 bzw. DE 197 22 062 A1 offenbart derart ausgebildet sein, dass über von dem Handgriff umgriffene Klemmelemente ein Lösen der Zugstange 13 aus dem Handgriff 50 zusätzlich verhindert und eine Verdrehsicherung bewirkt wird sowie dass eine Verriegelung des Schafts 1 im Handgriff 50 erfolgt. Wie in Fig. 6 dargestellt, umfasst der Handgriff 50 ferner ein feststehendes Teil 54, einen elektrischen Anschluss 55 zum Anschluss eines HF-Kabels und einen Druckknopf 56 zum Entriegeln des Schafts 1. Die über den elektrischen Anschluss 55 dem Instrument zugeführte HF-Spannung wird über den metallisch ausgeführten proximalen Abschnitt 14 und den distalen Abschnitt 15 der Zugstange 13 sowie ggf. über das Rohr 20 und die erste und die zweite Helix 23, 28 sowie das Rohr 25 zum Werkzeug 11 übertragen. Das dargestellte endoskopische Instrument ist ein monopolares elektrochirurgisches Instrument.

Zum Zerlegen des Instruments wird der schwenkbare Teil 53 des Handgriffs 50 in eine distale Endposition zum Lösen der Kugel 16 gebracht, der Druckknopf 56 betätigt und der Schaft 1 aus dem Handgriff 50 in distaler Richtung herausgezogen. Sodann wird der proximale Abschnitt 3 des Außenschafts 2 durch zunächst schraubenförmige und dann im Wesentlichen axiale Bewegung von dem distalen Abschnitt 4 des Außenschafts 2 gelöst und der proximale Abschnitt 14 der Zugstange 13 aus dem proximalen Abschnitt 3 des Außenschafts 2 herausgezogen. Sodann wird durch axiale Kraftausübung die Kugel 32 aus den Backen 41, 41' herauszogen. Die einzelnen Teile, nämlich der Handgriff 50, der proximale Abschnitt 3 des Außenschafts 2 und der distale Abschnitt 4 des Außenschafts 2 mit dem Werkzeug 11 und dem distalen Abschnitt 15 der Zugstange 13 haben jeweils eine deutlich geringere Länge als die Gesamtlänge des zusammengebauten endoskopischen Instruments und können in einem Sieb mit Standardabmessungen aufbewahrt und in einem Standard-Reinigungs- bzw. Sterilisationsapparat gereinigt bzw. sterilisiert werden. Eine Reinigung des distalen Abschnitts 4 des Außenschafts 2 mit dem damit verbundenen Werkzeug 11 und dem distalen Abschnitt 15 der Zugstange 13 ist dabei in üblicher Weise durch Anschließen an einen Adapter einer Reinigungseinrichtung zum Durchspülen des distalen Abschnitts 4 des Außenschafts 2 möglich, wobei der zwischen dem distalen Abschnitt 4 der Zugstange 13 und dem distalen Abschnitt 4 des Außenschafts 2 vorhandene Zwischenraum zur Durchleitung der Reinigungsflüssigkeit genutzt wird.

In den Figuren 7 bis 13 sind unterschiedliche Ausführungen der Verbindung zwischen dem Verbindungselement des distalen Abschnitts 15 der Zugstange 13 und dem Verbindungselement des proximalen Abschnitts 14 der Zugstange 13 dargestellt. Dabei können die jeweiligen Verbindungselemente wie nachfolgend beschrieben dem distalen Abschnitt 15 bzw. dem proximalen Abschnitt 14 zugeordnet sein, es ist aber auch eine umgekehrte Zuordnung möglich.

Gemäß Fig. 7 weist der distale Abschnitt 15 der Zugstange 13 an seinem proximalen Ende eine Kugel 32 auf, die wie in Fig. 3a und 3b dargestellt ausgebildet ist. Der Schnappmechanismus wird durch zwei Halbschalen 60, 60' gebildet, die ähnlich wie in Fig. 4a bis 4c gezeigt elastisch gegeneinander wirkend gelagert sind und die Kugel 32 kraftschlüssig halten. Wenn der proximale Abschnitt 14 der Zugstange 13 von den proximalen Abschnitt 3 des Außenschafts 2 umschlossen ist, wird ein Öffnen der beiden Halbschalen 60, 60' formschlüssig verhindert und die Kugel 32 somit formschlüssig gehalten.

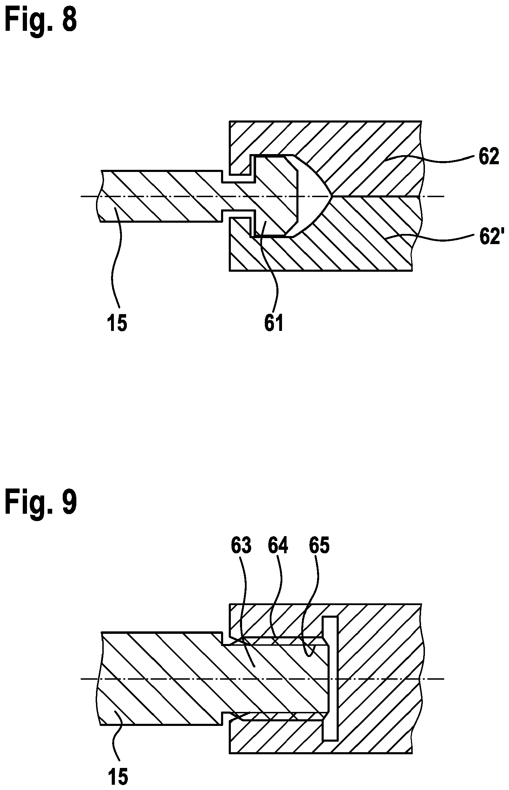

Wie in Fig. 8 gezeigt, kann das Verbindungselement des distalen Abschnitts 15 der Zugstange 13 auch eine andere Form aufweisen, beispielsweise als Platte 61 ausgebildet sein, die von den als Haken 62, 62' ausgebildeten Backen kraft- oder formschlüssig gegriffen und bei zusammengebautem Instrument formschlüssig gehalten wird.

Gemäß Fig. 9 kann ein proximaler Endabschnitt 63 des distalen Abschnitts 15 der Zugstange 13 ein Außengewinde 64 aufweisen, das mit einem entsprechenden Innengewinde 65 des distalen Endabschnitts des proximalen Abschnitts 14 des Zugstange 13 zusammenwirkt. Das Außengewinde 64 und das Innengewinde 65 weisen eine andere Richtung und eine andere Steigung auf als die erste und die zweite Helix 23, 28 des proximalen Abschnitts 3 bzw. des distalen Abschnitts 4 des Außenschafts 4 (s. Fig. 2a, 2b, 3a, 3b). Anstelle eines Gewindes kann ebenso eine bajonettartige Verbindung vorgesehen sein, wobei die Richtung zum Öffnen bzw. Schließen des Bajonetts ebenfalls gegenläufig zur Drehrichtung zum Öffnen bzw. Schließen der Verbindung der Helices 23, 28 gerichtet ist.

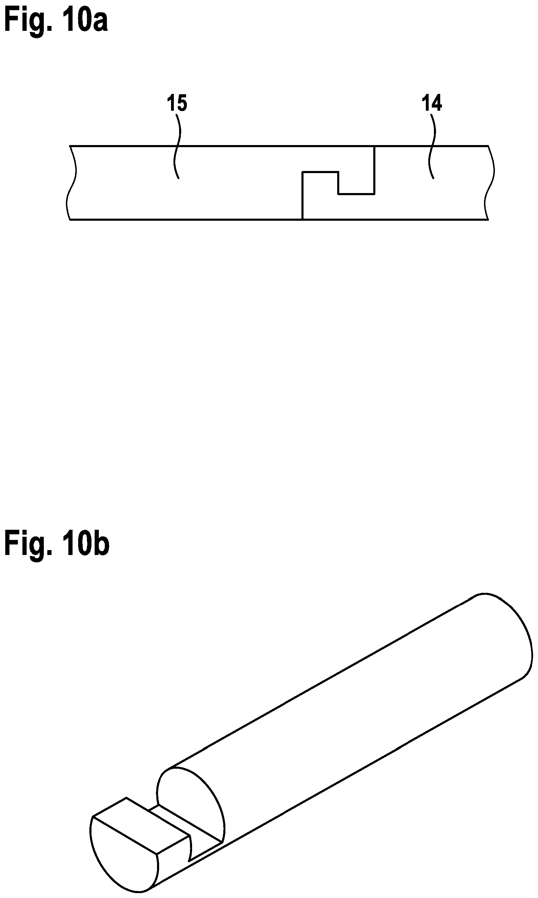

Wie in den Figuren 10a und 10b gezeigt, kann eine Verbindung durch komplementäre formschlüssige Ausbildung des proximalen Endes des distalen Abschnitts 15 mit dem distalen Ende des proximalen Abschnitts 14 der Zugstange 13 geschaffen werden. Die komplementären Verbindungselemente des distalen Abschnitts 14 und des proximalen Abschnitts 14 der Zugstange 13 können seitlich ineinander eingeschoben und dadurch miteinander verbunden sowie und durch seitliches Verschieben voneinander getrennt werden. Wenn die Zugstange 13 mit den beiden Verbindungselementen in den proximalen Abschnitt 3 des Außenschafts 2 eingeschoben ist, wird eine solche seitliche Verschiebung formschlüssig gesperrt und dadurch ein Lösen der Verbindung sicher verhindert. Die in den Figuren 10a und 10b gezeigten Verbindungselemente sind nicht axialsymmetrisch ausgebildet und ermöglichen somit eine verdrehgesicherte Verbindung des proximalen Abschnitts 14 mit dem distalen Abschnitt 15 der Zugstange 13.

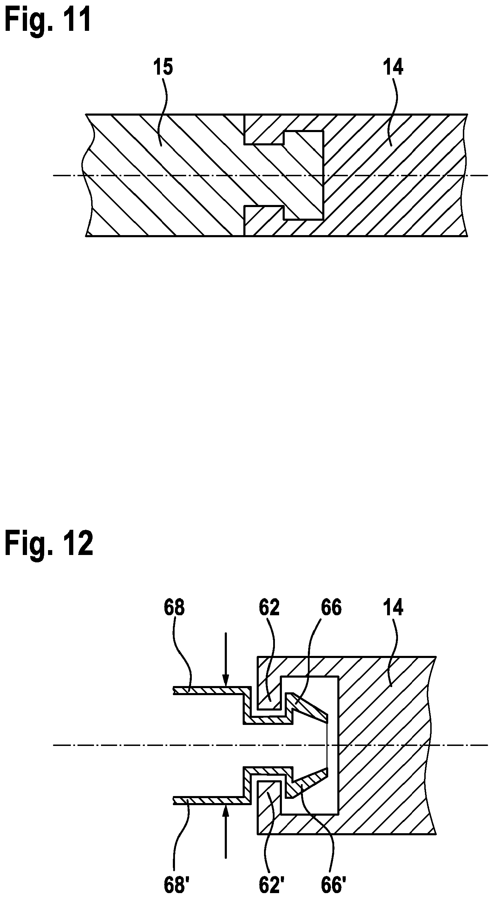

Eine weitere formschlüssige Ausbildung, bei der eine Verbindung durch seitliches Einschieben geschaffen wird, ist in Fig. 11 dargestellt. Auch diese Verbindungselemente sind nicht rotationssymmetrisch bezüglich der Längsachse der Zugstange 13 ausgebildet und ermöglichen es somit, eine axiale Verdrehung des distalen Abschnitts 15 gegenüber dem proximalen Abschnitt 14 der Zugstange 13 zu verhindern.

Gemäß Fig. 12 kann eine Verbindung durch als elastisch federnde Widerhaken 66, 66' ausgebildete, am proximalen Ende des distalen Abschnitts 15 der Zugstange 13 angeordnete Halteelemente geschaffen werden, die formschlüssig hinter axial nach innen weisende Vorsprünge 67, 67' am distalen Ende des proximalen Abschnitts 14 der Zugstange 13 greifen. Durch axiales Einschieben der Widerhaken 66, 66' hinter die Vorsprünge 67, 67' kann die Verbindung auf einfache Weise hergestellt werden. Durch seitliches Zusammendrücken der federnden Laschen 68, 68', die die Widerhaken 66, 66' tragen, kann die Verbindung wieder getrennt werden, wie in Fig. 12 durch die Pfeile angedeutet ist. Das durch die Laschen 68, 68' mit den Widerhaken 66, 66' gebildete Verbindungselement kann in einfacher Weise als geschlitztes Rohr ausgebildet sein.

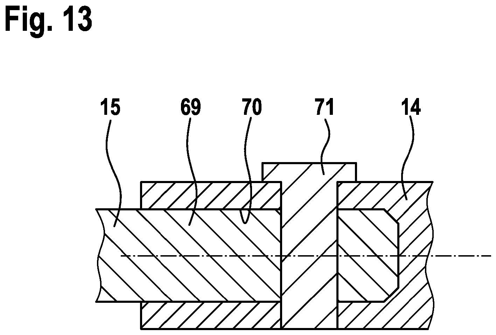

Wie in Fig. 13 gezeigt, kann bei einer weiteren Ausführungsform der Verbindungselemente ein zylindrischer proximaler Endabschnitt 69 des distalen Abschnitts 15 der Zugstange 13 in ein zylindrisches Sackloch 70 des proximalen Abschnitts 14 der Zugstange 13 in axialer Richtung eingeschoben und durch einen in Querrichtung einschiebbaren Stift 71 gegen Herausziehen gesichert werden. Der Stift 71 ist durch den über den proximalen Abschnitt 14 der Zugstange 13 geschobenen proximalen Abschnitt des Außenschafts 2 gegen Herausfallen gesichert. Der Stift 71 vermittelt ferner eine Verdrehsicherung der Verbindung.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 1: Schaft
- 2: Außenschaft
- 3: Proximaler Abschnitt
- 4: Distaler Abschnitt
- 5: Spülanschluss
- 6: Verbindungsmechanismus
- 7: Hülse
- 8: Anschlussstutzen
- 9: Lasche
- 10: Stopfen
- 11: Werkzeug
- 12, 12': Scherenblatt
- 13: Zugstange
- 14: Proximaler Abschnitt
- 15: Distaler Abschnitt
- 16: Kugel
- 17: Isolierhülse
- 20: Rohr
- 21: Schrumpfschlauch
- 22: Einsatz
- 23: Helix
- 25: Rohr
- 26: Schrumpfschlauch
- 27: Einsatz
- 28: Helix
- 30: Hebelmechanismus
- 31: Distaler Endbereich
- 32: Kugel
- 33: Stift
- 34: Stift
- 35: Schlitz
- 40: Schnappmechanismus
- 41, 41': Backe
- 42, 42': Lasche
- 43: Bohrung
- 44, 44': Schlitz
- 50: Handgriff
- 51: Kupplung
- 52: Drehrad
- 53: Schwenkbares Teil
- 54: Feststehendes Teil
- 55: Anschluss
- 56: Druckknopf
- 60, 60': Halbschalen
- 61: Platte
- 62, 62': Haken
- 63: Endabschnitt
- 64: Außengewinde
- 65: Innengewinde
- 66, 66': Widerhaken
- 67, 67': Vorsprung
- 68, 68': Lasche
- 69: Endabschnitt
- 70: Sackloch
- 71: Stift

## Patentansprüche

1. Schaft für ein starres endoskopisches Instrument, umfassend einen lang erstreckten Außenschaft (2) und ein innerhalb des Außenschafts (2) beweglich anordenbares lang erstrecktes Übertragungselement (13), **dadurch gekennzeichnet, dass** der Außenschaft (2) einen proximalen Abschnitt (3) und einen distalen Abschnitt (4) umfasst, die lösbar miteinander verbindbar sind,
wobei die beiden Abschnitte etwa gleich lang sind und jeweils etwa die halbe Länge des Außenschafts aufweisen.

2. Schaft nach Anspruch 1, **dadurch gekennzeichnet, dass** der proximale Abschnitt (3) des Außenschafts (2) an seinem distalen Ende und der distale Abschnitt (4) des Außenschafts (2) an seinem proximalen Ende jeweils ein Verbindungselement zur lösbaren Verbindung miteinander aufweisen, wobei die Verbindungselemente des proximalen Abschnitts (3) und des distalen Abschnitts (4) des Außenschafts (2) jeweils eine komplementär ineinandergreifende Helix (23, 28) aufweisen.

3. Schaft nach einem der vorhergehenden Ansprüche, **dadurch gekennzeichnet, dass** das Übertragungselement (13) einen proximalen Abschnitt (14) und einen distalen Abschnitt (15) umfasst, die lösbar miteinander verbindbar sind.

4. Schaft nach Anspruch 3, **dadurch gekennzeichnet, dass** der proximale Abschnitt (14) und der distale Abschnitt (15) des Übertragungselements (13) jeweils ein Verbindungselement zur formschlüssigen lösbaren Verbindung des proximalen Abschnitts (14) mit dem distalen Abschnitt (15) des Übertragungselements (13) aufweisen.

5. Schaft nach Anspruch 4, **dadurch gekennzeichnet, dass** die Verbindungselemente des proximalen Abschnitts (14) und des distalen Abschnitts (15) des Übertragungselements (13) derart miteinander verbindbar sind, dass bei einer Anordnung der Verbindungselemente im Außenschaft (2) der proximale Abschnitt (14) und der distale Abschnitt (15) des Übertragungselements (13) unlösbar miteinander verbunden sind.

6. Schaft nach Anspruch 4 oder 5, **dadurch gekennzeichnet, dass** die Verbindungselemente des proximalen Abschnitts (14) und des distalen Abschnitts (15) des Übertragungselements (13) zur Herstellung einer Schnappverbindung mit einem oder mehreren gelenkig oder elastisch federnd gelagerten Backen oder Widerhaken ausgebildet sind.

7. Schaft nach Anspruch 4 oder 5, **dadurch gekennzeichnet, dass** die Verbindungselemente des proximalen Abschnitts (14) und des distalen Abschnitts (15) des Übertragungselements (13) in einer Querrichtung ineinander einschiebbar sind.

8. Schaft nach Anspruch 4 oder 5, **dadurch gekennzeichnet, dass** die Verbindungselemente des proximalen Abschnitts (14) und des distalen Abschnitts (15) des Übertragungselements (13) durch Rotation um die Längsachse des Übertragungselements (13) miteinander verbindbar und voneinander trennbar sind.

9. Schaft nach einem der vorhergehenden Ansprüche, **dadurch gekennzeichnet, dass** der distale Abschnitt (15) und der proximale Abschnitt (14) des Übertragungselements (13) verdrehgesichert miteinander verbindbar sind und dass der distale Abschnitt (15) des Übertragungselements (13) verdrehgesichert mit dem distalen Abschnitt (4) des Außenschafts (2) verbunden oder verbindbar ist und der proximale Abschnitt (14) des Übertragungselements (13) verdrehgesichert mit dem proximalen Abschnitt (3) des Außenschafts (2) verbindbar ist.

10. Schaft nach Anspruch 9, **dadurch gekennzeichnet, dass** der proximale Abschnitt (14) des Übertragungselements (13) und der proximale Abschnitt (3) des Außenschafts (2) derart mit einem Handgriff (50) verbindbar sind, dass eine verdrehgesicherte Verbindung zwischen dem proximalen Abschnitt (14) des Übertragungselements (13) und dem proximalen Abschnitt (3) des Außenschafts (2) entsteht.

11. Schaft nach einem der vorhergehenden Ansprüche, **dadurch gekennzeichnet, dass** der Schaft (1) als Schaft eines elektrochirurgischen endoskopischen Instrumentes ausgebildet ist.

12. Starres endoskopisches Instrument mit einem lang erstreckten Schaft (1), einem an einem distalen Ende des Schafts (1) angeordneten Werkzeug (11) und einem an einem proximalen Ende des Schafts (1) angeordneten Handgriff (50), wobei der Schaft (1) einen lang erstreckten Außenschaft (2) und ein innerhalb des Außenschafts (2) beweglich angeordnetes lang erstrecktes Übertragungselement (13) umfasst und wobei das Werkzeug (11) und der Handgriff (50) mit dem Übertragungselement (13) und dem Außenschaft (2) derart verbunden sind, dass das Werkzeug (11) durch den Handgriff (50) mittels einer Bewegung des Übertragungselements (13) relativ zum Außenschaft (2) betätigbar ist, **dadurch gekennzeichnet, dass** der Schaft (1) nach einem der vorhergehenden Ansprüche ausgebildet ist.

## Claims

1. Shaft for a rigid endoscopic instrument, comprising an elongate outer shaft (2) and an elongate transmission element (13) that can be arranged movably inside the outer shaft (2), **characterized in that** the outer shaft (2) comprises a proximal portion (3) and a distal portion (4), which can be connected releasably to each other, wherein the two portions are approximately the same length and are each about half the length of the outer shaft.

2. Shaft according to Claim 1, **characterized in that** the proximal portion (3) of the outer shaft (2) has a connection element at its distal end, and the distal portion (4) of the outer shaft (2) has a connection element at its proximal end, said connection elements being provided for releasable connection to each other, wherein the connection elements of the proximal portion (3) and of the distal portion (4) of the outer shaft (2) have respective complementary helices (23, 28) engaging in each other.

3. Shaft according to one of the preceding claims, **characterized in that** the transmission element (13) comprises a proximal portion (14) and a distal portion (15), which can be connected releasably to each other.

4. Shaft according to Claim 3, **characterized in that** the proximal portion (14) and the distal portion (15) of the transmission element (13) each have a connection element for the form-fit releasable connection of the proximal portion (14) to the distal portion (15) of the transmission element (13).

5. Shaft according to Claim 4, **characterized in that** the connection elements of the proximal portion (14) and of the distal portion (15) of the transmission element (13) can be connected to each other in such a way that, when the connection elements are arranged in the outer shaft (2), the proximal portion (14) and the distal portion (15) of the transmission element (13) are connected non-releasably to each other.

6. Shaft according to Claim 4 or 5, **characterized in that** the connection elements of the proximal portion (14) and of the distal portion (15) of the transmission element (13) are designed for producing a snap-fit connection with one or more jaws or barbs mounted in an articulated or elastically resilient manner.

7. Shaft according to Claim 4 or 5, **characterized in that** the connection elements of the proximal portion (14) and of the distal portion (15) of the transmission element (13) can be pushed into each other in a transverse direction.

8. Shaft according to Claim 4 or 5, **characterized in that** the connection elements of the proximal portion (14) and of the distal portion (15) of the transmission element (13) can be connected to each other, and separated from each other, by rotation about the longitudinal axis of the transmission element (13).

9. Shaft according to one of the preceding claims, **characterized in that** the distal portion (15) and the proximal portion (14) of the transmission element (13) can be connected to each other in a manner secure against twisting, and **in that** the distal portion (15) of the transmission element (13) is connected or can be connected to the distal portion (4) of the outer shaft (2) in a manner secure against twisting, and the proximal portion (14) of the transmission element (13) can be connected to the proximal portion (3) of the outer shaft (2) in a manner secure against twisting.

10. Shaft according to Claim 9, **characterized in that** the proximal portion (14) of the transmission element (13) and the proximal portion (3) of the outer shaft (2) can be connected to a handle (50) in such a way that a connection secure against twisting is obtained between the proximal portion (14) of the transmission element (13) and the proximal portion (3) of the outer shaft (2).

11. Shaft according to one of the preceding claims, **characterized in that** the shaft (1) is designed as a shaft of an electrosurgical endoscopic instrument.

12. Rigid endoscopic instrument with an elongate shaft (1), a tool (11) arranged at a distal end of the shaft (1), and a handle (50) arranged at a proximal end of the shaft (1), wherein the shaft (1) comprises an elongate outer shaft (2) and an elongate transmission element (13) arranged movably inside the outer shaft (2), and wherein the tool (11) and the handle (50) are connected to the transmission element (13) and to the outer shaft (2) in such a way that the tool (11) can be actuated by the handle (50) by means of a movement of the transmission element (13) relative to the outer shaft (2), **characterized in that** the shaft (1) is designed according to one of the preceding claims.

## Revendications

1. Tige pour un instrument endoscopique rigide, comportant une tige extérieure étirée longitudinalement (2) et un élément de transmission étiré longitudinalement (13) pouvant être agencé de manière mobile à l'intérieur de la tige extérieure (2), **caractérisée en ce que** la tige extérieure (2) comporte une section proximale (3) et une section distale (4), qui peuvent être reliées l'une à l'autre de manière détachable,
les deux sections étant approximativement de même longueur et représentant chacune approximativement la moitié de la longueur de la tige extérieure.

2. Tige selon la revendication 1, **caractérisée en ce que** la section proximale (3) de la tige extérieure (2) à son extrémité distale et la section distale (4) de la tige extérieure (2) à son extrémité proximale comprennent chacune un élément de liaison pour la liaison détachable l'une à l'autre, les éléments de liaison de la section proximale (3) et de la section distale (4) de la tige extérieure (2) comprenant chacun une hélice (23, 28) entrant en prise mutuelle de manière complémentaire.

3. Tige selon l'une quelconque des revendications précédentes, **caractérisée en ce que** l'élément de transmission (13) comporte une section proximale (14) et une section distale (15), qui peuvent être reliées l'une à l'autre de manière détachable.

4. Tige selon la revendication 3, **caractérisée en ce que** la section proximale (14) et la section distale (15) de l'élément de transmission (13) comprennent chacune un élément de liaison pour la liaison détachable par accouplement de forme de la section proximale (14) à la section distale (15) de l'élément de transmission (13).

5. Tige selon la revendication 4, **caractérisée en ce que** les éléments de liaison de la section proximale (14) et de la section distale (15) de l'élément de transmission (13) peuvent être reliés l'un à l'autre de telle sorte que lors d'un agencement des éléments de liaison dans la tige extérieure (2), la section proximale (14) et la section distale (15) de l'élément de transmission (13) soient reliées l'une à l'autre de manière non détachable.

6. Tige selon la revendication 4 ou 5, **caractérisée en ce que** les éléments de liaison de la section proximale (14) et de la section distale (15) de l'élément de transmission (13) sont configurés pour la création d'une liaison encliquetable avec un ou plusieurs mors ou barbillons montés de manière articulée ou élastique.

7. Tige selon la revendication 4 ou 5, **caractérisée en ce que** les éléments de liaison de la section proximale (14) et de la section distale (15) de l'élément de transmission (13) sont insérables l'un dans l'autre dans une direction transversale.

8. Tige selon la revendication 4 ou 5, **caractérisée en ce que** les éléments de liaison de la section proximale (14) et de la section distale (15) de l'élément de transmission (13) peuvent être reliés l'un à l'autre et séparés l'un de l'autre par rotation autour de l'axe longitudinal de l'élément de transmission (13).

9. Tige selon l'une quelconque des revendications précédentes, **caractérisée en ce que** la section distale (15) et la section proximale (14) de l'élément de transmission (13) peuvent être reliées l'une à l'autre de manière immobilisée en rotation, et **en ce que** la section distale (15) de l'élément de transmission (13) est reliée ou peut être reliée de manière immobilisée en rotation à la section distale (4) de la tige extérieure (2), et la section proximale (14) de l'élément de transmission (13) peut être reliée de manière immobilisée en rotation à la section proximale (3) de la tige extérieure (2).

10. Tige selon la revendication 9, **caractérisée en ce que** la section proximale (14) de l'élément de transmission (13) et la section proximale (3) de la tige extérieure (2) peuvent être reliées avec une poignée (50) de telle sorte qu'une liaison immobilisée en rotation entre la section proximale (14) de l'élément de transmission (13) et la section proximale (3) de la tige extérieure (2) soit formée.

11. Tige selon l'une quelconque des revendications précédentes, **caractérisée en ce que** la tige (1) est configurée en tant que tige d'un instrument endoscopique électrochirurgical.

12. Instrument endoscopique rigide muni d'une tige étirée longitudinalement (1), d'un outil (11) agencé à une extrémité distale de la tige (1) et d'une poignée (50) agencée à une extrémité proximale de la tige (1), la tige (1) comportant une tige extérieure étirée longitudinalement (2) et un élément de transmission étiré longitudinalement (13) agencé de manière mobile à l'intérieur de la tige extérieure (2), et l'outil (11) et la poignée (50) étant reliés à l'élément de transmission (13) et à la tige extérieure (2) de telle sorte que l'outil (11) puisse être actionné par la poignée (50) au moyen d'un mouvement de l'élément de transmission (13) relativement à la tige extérieure (2), **caractérisé en ce que** la tige (1) est configurée selon l'une quelconque des revendications précédentes.
